# EUROPEAN PATENT SPECIFICATION

(11) **EP 1 027 433 B1**
(45) Date of publication and mention of the grant of the patent: **03.09.2003**
(21) Application number: 98932168.2
(22) Date of filing: 09.06.1998
(51) Int. Cl.: C12N 15/12, C12N 15/00, C07K 14/47, A01K 67/027, C12N 5/10

(54) **HUMAN APP OR A4CT SEQUENCES ENCODING THE MUTATION I45F**
FÜR DIE I45F-MUTATION KODIERENDE HUMAN APP ODER A4CT SEQUENZEN
SEQUENCES APP OU A4CT HUMAINES CODANT LA MUTATION 145F

(30) Priority: 18.06.1997 GB 9712849
(43) Date of publication of application: 16.08.2000
(73) Proprietor: SmithKline Beecham Pharma GmbH & Co. KG, 80804 München (DE); SmithKline Beecham (Australia) Pty Ltd, Dandenong, Victoria 3175 (AU); SMITHKLINE BEECHAM PLC, Brentford, Middlesex TW8 9GS (GB)
(72) Inventor: LICHTENTHALER, Stefan, University of Heidelberg, D-69120 Heidelberg (DE); BEYREUTHER, Konrad, University of Heidelberg, D-69120 Heidelberg (DE); MASTERS, Colin, Louis, Parkville, VIC 3052 (AU); BROWN, Frank, SmithKline Beecham Pharmaceuticals, Harlow, Essex CM19 5AW (GB); ALLSOP, David, SmithKline Beecham Pharmaceuticals, Harlow, Essex CM19 5AW (GB)
(74) Representative: Valentine, Jill Barbara
(86) International application number: EP9803686
(87) International publication number: WO98058060

(56) References cited:
- WO-A-98/03643
- LICHTENTHALER S ET AL: "MUTATIONS NEAR THE C-TERMINUS OF BETAA4 INFLUENCE THE GAMMA-CLEAVAGE OF A4CT (C99) IN COS7 CELLS" NEUROBIOLOGY OF AGING, vol. 17, no. 4, SUPPL, 19 July 1996, page S130 XP002056699
- LICHTENTHALER S F ET AL: "MUTATIONS IN THE TRANSMEMBRANE DOMAIN OF APP ALTERING GAMMA SECRETASE SPECIFICITY" BIOCHEMISTRY, vol. 36, no. 49, 9 December 1997, pages 15396-15403, XP002056700

## Description

This invention relates to modified amyloid precursor proteins and their use in the production of transgenic animals.

The main protein component of the amyloid plaques found in the brain of Alzheimer's disease (AD) patients is βA4, a 4 kDa peptide consisting of mainly forty and forty-two residues (βA4₁₋₄₀, βA4₁₋₄₂) being derived from the amyloid precursor protein (APP). APP can exist in multiple forms generated by alternative mRNA splicing. The first form of APP identified by Kang, J., et al., (ref 37) from a foetal brain cDNA library contained 695 amino acids (so-called APP695). This includes a 17 residue signal peptide sequence for transport of the protein into the endoplasmic reticulum. Subsequently, a number of slightly longer cDNA clones were isolated by other workers. The 751 amino acid APP sequence (APP751) described by Ponte, P., et al., (ref 38) contained an additional 57-amino acid insert encoding a Kunitz-type serine proteinase inhibitor (KPI). Kitaguchi, N., et al., (ref 39) identified another precursor of 770 amino acids (APP770) with both the KPI sequence and an additional 19 amino acid insert. These isoforms of APP arise as a result of alternative splicing of exons 7 and 8 during transcription of the APP gene. Additional isoforms generated by alternative splicing of exon 15 have also been detected (ref 40).

APP can be cleaved at the N-terminus of βA4 by an enzyme called β-secretase to generate soluble APP and the C-terminal fragment A4CT (C99). This 99 residue long membrane protein A4CT (ref. 1) which is the direct precursor for βA4 contains the entire βA4 domain, the membrane domain and the cytoplasmic tail of APP. Alternative processing of APP in a post-Golgi-compartment by a protease termed α-secretase leads to the cleavage of APP within the βA4 domain yielding secretory APP and the transmembrane fragment p3CT which is the direct precursor for p3.

Human APP herein refers to all isoforms including the 695 form.

Both C-terminal fragments of APP, A4CT and p3CT, are cleaved within the membrane domain by a γ-cleavage activity, thereby releasing βA4 and p3 into the medium (refs. 2, 3). In cells expressing wild type APP the site of γ-cleavage is mainly the peptide bond Val(40)-Ile(41)of A4CT and to a minor extent the bond Ala(42)-Thr(43). In cells expressing APP with the Familial AD linked mutations at Val 717 (based on APP₇₇₀. Val 46 of A4CT) an increased γ-cleavage occurs behind Val(42), thus producing larger amounts of βA4₁₋₄₂ (ref. 4).

Transgenic mice expressing A4CT have been produced using the human APP promoter (ref. 5), the human thy-1 promoter (ref. 6) and the JC viral early region promoter (ref. 7). Transgenic mice have also been produced expressing amino acids 591-695 of APP695 (C-terminal 105 amino acids of APP) under control of the human neurofilament NF-L transcriptional regulatory sequences (ref . 41). Numerous promoters have been used inconjunction with the full length APP cDNA (refs. 12, 13, 41, 42, 43, 46). Generation of transgenic mammals bearing APP derived DNA sequences are also described in WO93/14200 (TSI Corporation), WO91/19810 (California Biotechnology Inc), WO93/02189 (University of Calfornia), WO89/00689, WO92/06187 (The Upjohn Company), EP0451700 (Miles Inc.), WO92/1306 (Imperial College of Science Technology and Medicine) and WO89/06689 (McClean Hospital Corporation). In some instances, transgenic mice bearing human mutant APP have been crossed with presenilin-1 transgenics to produce 'double mutant' mice (refs. 44, 45).

Results obtained depend upon the source of the promoter and the protein coding sequence used. However in all cases described to date the nature of the APP-immunoreactive deposits did not resemble the clinical situation and, with the exception of the model described in references 13. 42, 44 and 45 such transgenic animals have not been found to be faithful model systems for Alzheimer's disease.

WO98/03643 (published 29 January 1998) and refs. 36 and 47 disclose constructs comprising mutant human APP or A4CT DNA sequences.

The βA4₁₋₄₂ peptide is the major subunit of amorphous and neuritic plaques in Alzheimer's disease.

Applicants have found that recombinant cells expressing A4CT carrying certain mutations in the A4CT amino acid sequence lead to a higher ratio of βA4₁₋₄₂/βA4₁₋₄₀ than wild type and such mutant proteins and coding DNA are therefore useful in the production of transgenic animals developing amyloid plaques as a model of Alzhemer's disease. It will be understood that references herein to A4CT, βA4₁₋₄₂ and βA4₁₋₄₀ include all N-terminal variants produced by alternative cleavage during processing.

According to the present invention there is provided a non-human transgenic mammal whose cells contain a construct comprising a human APP or A4CT DNA sequence encoding the mutation I45F (numbering relative to A4CT).

In a preferred aspect the DNA sequence is operably linked to a promoter sequence.

In a further aspect the construct further encodes an insertion in the transmembrane domain (residues 43 to 52) of a sequence of 1 to 10 amino acid residues; (numbering relative to A4CT) operably linked to a promoter sequence.

The insertion is preferably located between residues 42 and 53, more preferably between 46 and 53. In a preferred embodiment the insertion is located between T48 and L49. The residues for insertion are preferably selected from F, I, G, Y, L, A, P, W, M, S, T, N and Q. The insertion is preferably 2 to 6 residues long. In a preferred embodiment the insertion is LV.

In a further preferred aspect the construct additionally encodes a mutation selected from:
V46F, V46I, V46G, V46Y, V46L, V46A, V46P, V46W, V46M, V46S, V46T, V46N or V46Q. In a preferred embodiment the additional mutation is V46F.

In a preferred embodiment the construct comprises an A4CT DNA sequence and further encodes the APP signal sequence (APP residues 1 to 17) immediately upstream of the A4CT DNA sequence. Hydrophobic residue inserts such as LeuGlu or Met are necessary for processing of A4CT to βA4 and should preferably be included between the signal peptide and A4CT coding regions and will remain attached to the processed A4CT.

In a further preferred embodiment the APP is full length APP695.

The invention also relates to mammalian cells expressing the construct and to the DNA construct itself and vectors containing it.

Generation of transgenic non-human mammals of the invention may be carried out conventionally, for example as described in WO93/14200, WO91/19810, WO93/02189, WO89/00689, WO92/06187, EP0451700, WO92/13069 and WO89/06689.

The APP or A4CT coding DNA is obtained by probing a human cDNA library. Mutations may be introduced by site-directed mutagenesis or during construction of the coding DNA from appropriate fragments.

Suitable promoters for use in the present invention include: Human APP (ref. 5); rat neuron specific enolase (neurons) (ref. 18); human β actin (ref. 19); human PDGFβ (ref. 20); mouse Thy 1 (ref. 21); mouse Prion protein promoter (PrP) (ref. 14); Syrian hamster Prior protein promoter (ref. 35); rat synapsin 1 (brain) (ref. 22); human FMR1 (brain) (ref. 23); human neurofilament low (ref. 24), middle (brain) (ref. 25); NEX-1 (brain) (ref. 26); mouseAPLP2 (brain) (ref. 27); rat alpha tubulin (ref. 28); mouse transferrin (ref. 29); mouse HMGCR (3-hydroxy-3-methylglutaryl coenzyme A reductase, oligodendrocytes) (ref. 30) and mouse myelin basic protein (ref. 31).

A tetracycline-inducible system may also be used, which has the advantage of regulating the gene expression (induction/repression) (refs. 33, 32). This system uses two constructs: a minimal promoter (PhCMV*-1) fused to seven tetracyclic operator sequences and the cDNA in question; and a trangene containing the tetracycline-controlled trap-activator protein (tTA) coding sequence under the control of a promoter, for example taken from the above list. Each construct is used to generate a transgenic mouse. Crossing the two homozygous mice generates a double transgenic line which expresses the tTA according to the chosen promoter. This tTA induces expression of the cDNA by activating the PhCMV*-1, but only in the absence of tetracycline. In the presence of tetracycline there is only basal expression.

A preferred promoter is the mouse Prion protein promoter (ref. 14) or hamster Prion protein promoter (ref. 35).

The construct is prepared by conventional recombinant DNA techniques (ref. 10).

The transgenic non-human mammal is produced by conventional techniques (refs. 8, 15, 16, 17).

In one aspect, the transgenic non-human mammal is produced by introduction of the construct into an embryo, insertion of the embryo into a surrogate mother and allowing the embryo to develop to term.

The construct is prepared for transfer to the host animal by cleavage of vector containing the construct and purification of the DNA (ref. 8)

The transfer is carried out conventionally preferably using microinjection as described in detail in reference 8.

In an alternative aspect the transgenic non-human mammal is produced by introduction of the construct into embryonic stem cells by conventional methods such as calcium phosphate/DNA precipitation, direct injection or electroporation (ref. 9) followed by injection of the transformed cells into blastocytes and insertion of the resulting embryo into a surrogate mother as described above.

Transgenic non-human animals are identified by DNA analyis using Southern blot and PCR to detect founder animals.

The transgenic non-human mammal is preferably a rodent such as rat or mouse, more preferably a mouse.

Mammalian cells expressing the construct may be prepared by conventional methods.

Host cells are genetically engineered (transduced or transformed or transfected) with the vectors of this invention which may be, for example, a cloning vector or an expression vector. The vector may be, for example, in the form of a plasmid, a viral particle, a phage, etc. The engineered host cells can be cultured in conventional nutrient media modified as appropriate for activating promoters, selecting transformants or amplifying the human genes. The culture conditions, such as temperature, pH and the like will be apparent to the ordinarily skilled artisan.

Various mammalian cell culture systems can be employed to express recombinant protein. Examples of mammalian expression systems include the COS-7 lines of monkey kidney fibroblasts, described by Gluzman, Cell, 23:175 (1981), and other cell lines capable of expressing a compatible vector, for example, the SH-SY5Y, CHO and HeLa cell lines.

The selection of an appropriate host is deemed to be within the scope of those skilled in the art from the teachings herein.

Mammalian expression vectors will comprise an origin of replication, a suitable promoter and enhancer, and also any necessary ribosome binding sites, polyadenylation site, splice donor and acceptor sites, transcriptional termination sequences, and 5' flanking nontranscribed sequences. DNA sequences derived from the SV40 splice, and polyadenylation sites may be used to provide the required nontranscribed genetic elements.

In addition, the expression vectors preferably contain one or more selectable marker genes to provide a phenotypic trait for selection of transformed host cells such as hygromycin or neomycin resistance for eukaryotic cell culture.

The appropriate DNA sequence may be inserted into the vector by a variety of procedures. In general, the DNA sequence is inserted into an appropriate restriction endonuclease site(s) by procedures known in the art. Such procedures and others are deemed to be within the scope of those skilled in the art.

The DNA sequence in the expression vector is operatively linked to an appropriate expression control sequence(s) (promoter) to direct mRNA synthesis. Examples of such promoters include the CMV promoter, pCEP4 (Invitrogen) and other promoters known to control expression of genes in eukaryotic cells or their viruses and replicable and viable in the host.

Introduction of the construct into the host cell can be effected by calcium phosphate transfection, lipofectin-mediated transfection, or electroporation. (Davis, L., Dibner, M., Battey, I., Basic Methods in Molecular Biology, (1986)).

The transgenic mammal or cells of the invention may be used to screen for drugs which inhibit deposit of βA4 by administering test drug to the mammal or cell culture medium and observing changes in APP expression and processing, histopathology and/or behavioural changes. The invention extends to such method of screening.

Suitable techniques for making such observations are described in WO93/14200.

### Examples

### SPA4CT I45F (SEQ ID NOs: 1 and 2)

### SPA4CT V46F (FAD mutation).

The above constructs with sequences given in Table 2 were prepared by site directed mutagenesis of wtSPA4CT (ref.1):
wtSPA4CT consists of the 17 amino acid long signal peptide of APP followed by two additional amino acids of APP 695 (Leu and Glu) and then continuing with the βA4 sequence and the whole C-terminal domain and the mutagenesis was carried out in vector pSP65/SPA4CT (ref. 11) for V46F mutation and pBS/SPA4CT rev for I45F mutation. pBS/SPA4CT rev was obtained by cloning the KpnI/Nhe fragment of pCEP/SPA4CT (ref. 2) in the pBS/SPC99 vector (ref. 34) that was digested with KpnI/XbaI.

The constructs were inserted into pCEP vector (ref. 2) and were stably transfected into COS7 cells.

### APP695 I45F (SEQ ID NOs: 3 and 4)

The above construct with sequences given in Table 2 was prepared by site directed mutagenesis of full length wild type APP695 followed by insertion into the SalI site of cosmid cosSHa.Tet (ref. 35), such that the tetracycline resistance gene in the cosmid was replaced by the 145F mutant form of APP695 downstream of the hamster Prion promoter.

### Biological activity

### Measurement of βA4 in the conditioned medium

Stably transfected COS7 cells were metabolically labeled over night in methionine free MEM-medium containing 10% FCS and 133 µCi/ml ³⁵S-methionine. βA4 and A4CT were immunoprecipated, separated on a 10% Tris-Tricine gel and quantified by phosphorimaging. The following antibodies were used:
G2-10 (monoclonal) against synthetic peptide βA4 33-40 for the immunoprecipitation of βA4ₙ₋₄₀,
G2-11 (monoclonal) against synthetic peptide βA4 35-42 for the immunoprecipitation of βA4ₙ₋₄₂,
692 (polyclonal rabbit serum) against synthetic peptide βA4 1-40 for the immunoprecipitation of βA4.
n-40 and n-42 stand for peptides with a defined C-terminus (ie residue 40 or 42 respectively of βA4) but allowing for possible N-terminal homogeneity. The full length βA4 forms produced by the particular constructs described herein contain Leu, Glu at positions -2, -1.

### Detection of A4CT in the cell lysate

The stably transfected COS7 cells were metabolically labeled for 10 min in methionine free MEM-medium containing 133 µCi/ml ³⁵S-methionine. In the cell lysate A4CT was immunoprecipitated with polyclonal antibody against A4CT (ref. 2), separated on a 10% Tris-Tricine gel and quantified by phosphorimaging.

### Results

### Expression of A4CT

All constructs (wt SPA4CT and mutated SPA4CT) were expressed in similar amounts. The signal peptide of SPA4CT was completely removed leading to Leu-Glu-A4CT.

### Release of βA4

All A4CT constructs were processed to βA4 and produce similar amounts of βA4.

### Generation of βA4₁₋₄₂ and βA4₁₋₄₀

For all the constructs both βA4 species βA4₁₋₄₂ and βA4₁₋₄₀ were released. The ratio βA4₁₋₄₂/βA4₁₋₄₀ was determined and the results are shown in Table 1.

**Table 1**

| **SPA4CT** | **bA4**_{**1-42**}**/bA4**_{**1-40**} | **increase of bA4**_{**1-42**}**/bA4**_{**1-40**} **relative to wt SPA4CT by factor** | **significance p (student's t test)** |
|---|---|---|---|
| wildtype | 4.7 ± 1.3 | 1.0 | - |
| V46F | 17.1 ± 4.8 | 3.6 | < 0.001 |
| I45F | 161.9 ± 5.7 | 33.9 | <0.001 |

### Conclusions

The FAD linked mutation Val(717)Phe (Val(46)Phe of A4CT) is known to lead to a higher ratio of βA4₁₋₄₂/βA4₁₋₄₀ for both SPA4CT and APP compared with the wildtype proteins.

The above results demonstrate that SPA4CT expressing cells (COS7) generate the same βA4 species (βA4₁₋₄₀ and βA4₁₋₄₂) as APP expressing cells. This suggests that the mechanism of βA4 generation is the same in APP and SPA4CT expressing cells.

The mutations near the C-terminus of βA4 are able to influence the γ-cleavage site, whereas the overall amount of generated βA4 as well as the ratio of βA4/p3 remain unchanged.

The mutant I45F has an increased ratio βA4₁₋₄₂/βA4₁₋₄₀ relative to wt.

### Generation of transgenic mice expressing mutant SPA4CT or APP695

The above described constructs which produce enhanced production of βA4₁₋₄₂.relative to βA4₁₋₄₀ are useful for the generation of transgenic mice developing amyloid plaques.

### Example 1

The mutant SPA4CT construct driven by the mouse Prion protein promoter (ref. 14) was used to transform a mouse by the following procedures:
The construct is prepared and purified.
Female mice are induced to superovulate and embryos are recovered.
DNA is microinjected into the pronucleus of embryos.
Embryos are transferred into pseudopregnant mice (female mice previously paired with vasectomised males).
Embryos are developed and mice are born.
Founder mice are identified by Southern blot and PCR and bred on.

The mice lines were as follows:
Donor mice (embryos for pronucleus injection): DBA2
Acceptor mice: NMRI
Mice for further breeding: C57B1/6

### Example 2

The full length human APP695 gene carrying the I45F mutation driven by the hamster Prion promotor (ref. 35) was used to generate a mouse also by the procedures of Example 1.
The mice lines were as follows;
Donor mice : C57B1/6 inbred mice
Recipient mice: [C57B1/6 x CBA]F1
Mice for further breeding: C57B1/6

A modification was made to the conventional techniques for generating transgenic animals by microinjection. This modification was to utilize a particularly shaped microinjection needle. The needle is made from the standard borosilicate glass but with a larger tip size and a more splintered shape to that generally used. This was to overcome the slightly larger size of the microinjection fragment used to generate the transgenic mice (approx. 40 kilobases over the standard 1-10 kilobases).

Hybrid [C57B1/6 x CBA] strain may alternatively be used instead of C57B1/6.

### Screening of drugs using transgenic mice

The transgenic mice described above may be used to screen for potential activity of test drugs in the treatment of Alzheimer's disease.

APP expression and processing may be examined using detection of mRNA by Northern blots and detection of polypeptides using polyclonal and monoclonal antibodies that are specific to the terminal regions of the target peptides.

Histopathological observations may be made using immunohistological techniques to permit identification of amyloid plaques and in situ hybridisation using labelled probes to target mRNA.

Observation of behavioural changes may employ conventional tests used to assess learning and memory deficits.

### References

1. Dyrks T., Weidemann A., Multhaup G., Salbaum J. M., Lemaire H.-G., Kang J., Müller-Hill B., Masters C. L., Beyreuther K. (1988), EMBO J. 7, 949-957.
2. Dyrks T., Dyrks E., Mönning U., Urmoneit B., Turner J., Beyreuther K. (1993), FEBS Letters 335, 89-93.
3. Haass C., Hung A. Y., Schlossmacher M. G., Teplow D. B., Selkoe D. J. (1993), JBC 268, 3021-3024.
4. Suzuki N.; Cheung T. T.; Cai X. D.; Odaka A.; Otvos L. Jr.; Eckman C.; Golde T. E., Younkin S. G. (1994), Science 264, 1336-1340.
5. Wirak D. O., et al (1991) Science 253, 323-325.
6. Kawabata S. (1991) Nature 354 476-478.
7. Sandhu F.A. (1991) JBC 266 21331-21334.
8. Hogan et al., 'Manipulating the mouse embryo', Cold Spring Harbor Laboratory, Cold Spring Harbor (1994).
9. Teratocarcinomas and embryonic stem cells, a practical approach' ed. E. J. Robertson, IRL Press, 1987.
10. Maniatis et al., 'Molecular Cloning, a Laboratory Manual', Cold Spring Harbor Laboratory, Cold Spring Harbor (1989).
11. Dyrks T., Dyrks E., Masters C., Beyreuther K. (1992), FEBS Letters 309 20-24.
12. Greenberg B.D. et al., Neurobiology of Aging (1996) 17(2) 153-171.
13. Games et al., Nature (1995) 373, 523-527.
14. M. Fischer et al., EMBO J. (1996) 15(6) 1255-64.
15. Transgenic animal technology', a Laboratory Handbook ed. C.A. Pinkert (Academic Press Inc 1994).
16. Transgenesis Techniques' Principles and Protocols ed. D. Murphy and D.A. Carter (Humana Press, Totowa, New Jersey 1993).
17. Teratocarcinomas and Embryonic Stem Cells', a Practical Approach ed. E.J. Robertson (IRL Press 1987).
18. Forss-Petter et al., (1990) Neuron 5;197.
19. Ray et al., Genes and Development 5;2265-2273 (1991).
20. Sasahara et al., Cell 64;217-227 (1991).
21. Ingraham etal., Mol. Cell. Biol. 6(8) 2923-31 (1986).
22. Howland et al., Neurobiol. Aging 16(4) 685-99, (1995).
23. Hergersberg et al., Hum. Mol. Genet. 4(3) 359-66 (1995).
24. Thomas et al., J. Virol. 68(11) 7099-107 (1994).
25. Tu et al., J.Cell. Biol. 129(6) 1629-40 (1995).
26. Bartholoma et al., Mech. Dev. 48(3), 217-8 (1994).
27. Kock et al., J. Biol. Chem. 270(43) 25475-80 (1995).
28. Gloster et al., J. Neurosci. 14(12), 7319-30 (1994).
29. Thiesen et al., Mol. Cell. Biol. 13(12) 7666-76 (1993).
30. Duhamel-Clerin et al., Glia 11(1) 35-46 (1994).
31. Readhead et al., Cell 48;703-712 (1987).
32. Baron et al., Nucl. Acids Res. 23(17) 3605-6 (1995).
33. Furth et al., PNAS USA 91, 9302-9306 (1994).
34. Tienari et al., EMBO J 15, 5218-5229 (1996).
35. Scott et al., Prot. Sci. 1, 986-997 (1992).
36. Lichtenthaler et al., Neurobiology of Aging 17(4) supp, s130-s131 abstract 524.
37. Kang et al., Nature (1987) 325:733-736.
38. Ponte et al., Nature (1998) 331: 525-527.
39. Kitaguchi et al., Nature (1988) 331:530-532.
40. Sandbrink, et al., J. Biol. Chem. (1994) 269: 1510-1517.
41. Nalbantoglu et al., Nature (1997) 387: 500-505.
42. Hsiao et al., Science (1996) 274, 99-103.
43. Sturchler-Pierrat et al., Proc. Natl. Acad. Sci. USA (1997) 94: 13287-13292.
44. Borchelt et al., Neuron (1997) 19: 939-945.
45. Holcomb et al., Nature Med. (1998) 4: 97-100
46. Duff, Trend Neurosci. (1997) 20: 279-80
47. Lichtenthaler et al., Biochemistry (1997) 36: 15396-15403

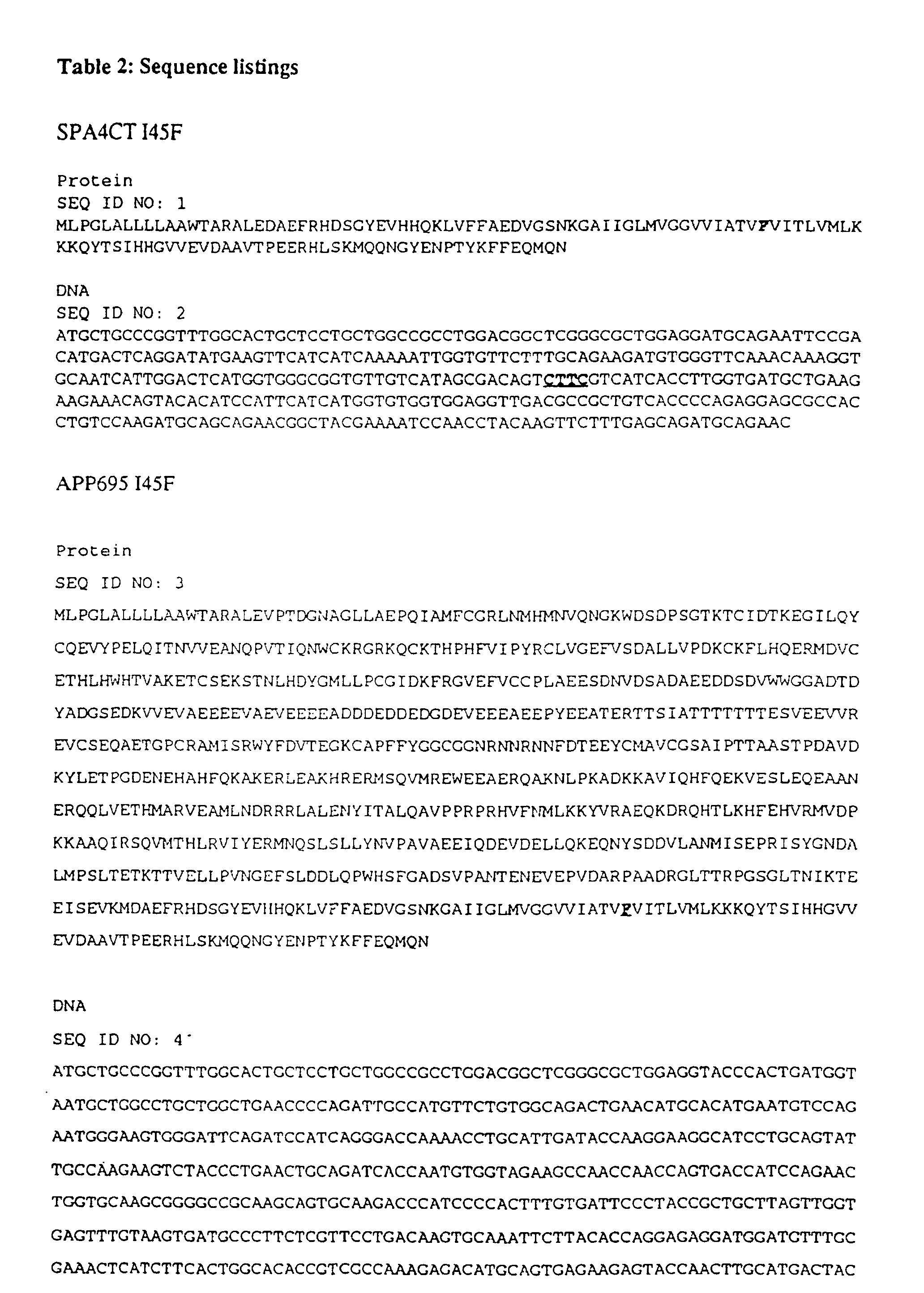

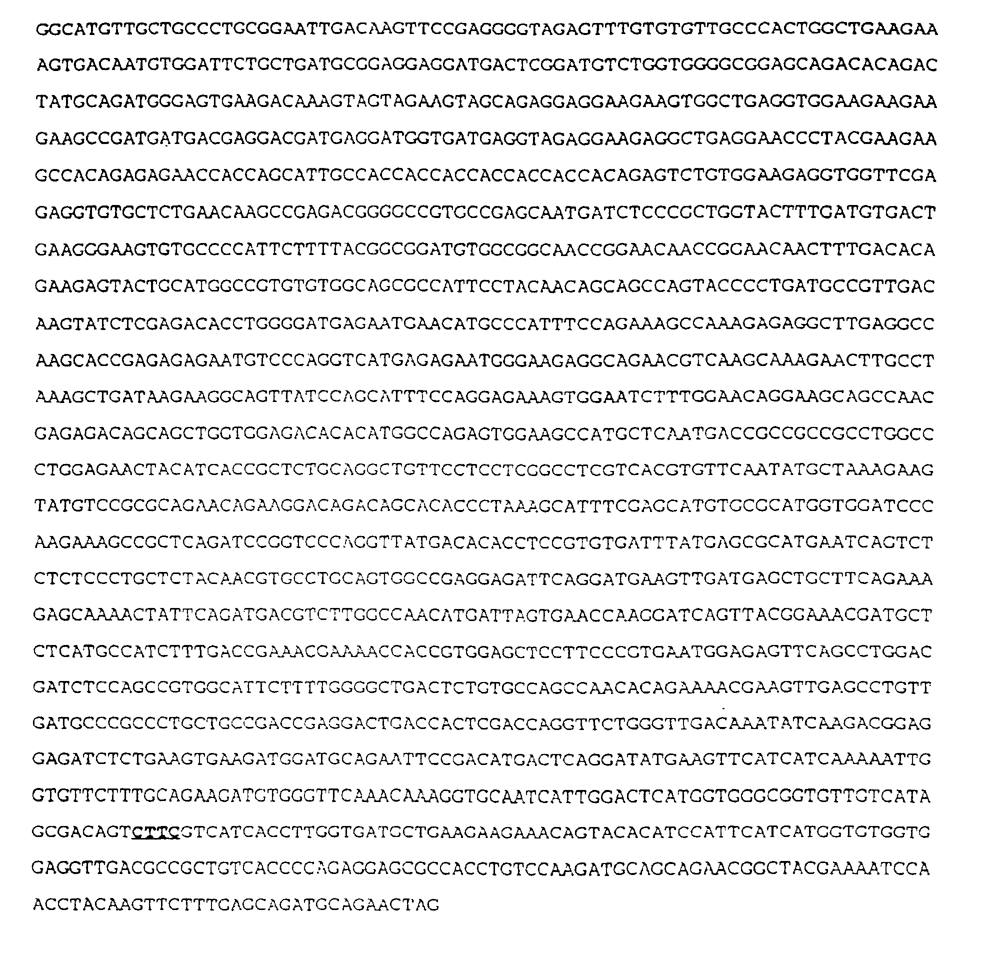

## Claims

1. A construct comprising a DNA sequence encoding either human Amyloid Precursor Protein (APP) a A4CT, wherein the DNA sequence encodes the mutation 145 F (membering relative to A4CT).

2. A construct according to claim 1 where the DNA sequence is operably linked to a promoter sequence.

3. A construct according to claim 1 wherein the construct further encodes an insertion in the transmembrane domain of a sequence of 1 to 10 amino acid residudes.

4. A construct according to claim 3 wherein the insertion is located between T48 and L49.

5. A construct according to claim 3 or 4 wherein the residues for insertion are selected from F, I, G, Y, L, A, P, W, M, S, T, N and Q.

6. A construct according to any of claims 3 to 5 wherein the insertion is 2 to 6 residues long.

7. A construct according to claim 6 wherein the insertion is LV.

8. A construct according to any preceding claim wherein the construct additionally encodes a muation selected from: V46F. V46I, V46G, V46Y, V46L, V46A, V46P, V46W, V46M, V46S, V46T, V46N, or V46Q.

9. A construct according to any preceding claim wherein the construct comprises an A4CT DNA sequence and further encodes the Amyloid Precursor Protein signal sequence (APP residues 1 to 17) immediately upstream of the A4CT DNA sequence together with hydrophobic residue inserts between the signal peptide and A4CT coding regions.

10. A construct according to any preceding claim comprising a mammalian promoter selected from Human APP (Amyloid Precursor Protein) rat neuron specific enolase; human β actin; human PDGFβ (platelet Derived Growth Factor β); mouse Thy 1; mouse prion protein promoter, Syrian hamster Prion protein promoter; rat synapsin 1; human FMR1 (Fragile X Mental Retardation 1); human neurofilament low, middle; NEX-1; mouse APLP2 (β Amyloid Precursor Like Protein 2); rat alpha tubulin; mouse transferrin; mouse HMGCR (3-hydroxy-3-methylglutaryl coenxyme A reductase) and mouse myelin basic protein.

11. A construct according to claim 1 comprising the sequence SPA4CT 145F (SEQ ID NO:2)

12. A construct according to claim 11 wherein the DNA sequence is operably linked to mouse Prion protein promoter.

13. A construct according to claim 1 comprising the sequence APP695 145F (SEQ ID NO:4)

14. A construct according to claim 13 wherein the DNA sequence is operably linked to Syrian hamster Priors protein promoter.

15. A non-human transgenic mammal whose cells contain a construct according to any preceding claim.

16. A transgenic mammal according to claim 15 which is a rodent.

17. A transgenic mammal according to claim 16 which is a mouse.

18. A mammalian host cell expressing the construct of any of daims 1 to 14.

19. A vector containing the construct of any of claims 1 to 14.

20. A method of screening for drugs which inhibit deposit of βA4 by administering a test drug to the transgenic mammal of claim 15, 16 or 17 or cell culture medium containing the mammalian host cell of claim 18 and observing changes in APP expression and processing, histopathology and/or behavioural changes.

## Patentansprüche

1. Konstrukt, das eine DNA-Sequenz umfasst, die entweder humanes Amyloid-Vorläuferprotein (APP) oder A4CT codiert, worin die DNA-Sequenz die Mutation I45F codiert (Numerierung relativ zu A4CT).

2. Konstrukt gemäss Anspruch 1, worin die DNA-Sequenz funktionsfähig mit einer Promotor-Sequenz verknüpft ist.

3. Konstrukt gemäss Anspruch 1, worin das Konstrukt ferner eine Insertion in der Transmembrandomäne einer Sequenz von 1 bis 10 Aminosäureresten codiert.

4. Konstrukt gemäss Anspruch 3, worin die Insertion sich zwischen T48 und L49 befindet.

5. Konstrukt gemäss Anspruch 3 oder 4, worin die Reste zur Insertion ausgewählt sind aus F, I, G, Y, L, A, P, W, M, S, T, N und Q.

6. Konstrukt gemäss einem der Ansprüche 3 bis 5, worin die Insertion 2 bis 6 Reste lang ist.

7. Konstrukt gemäss Anspruch 6, worin die Insertion LV ist.

8. Konstrukt gemäss jedem vorhergehenden Anspruch, worin das Konstrukt zusätzlich eine aus V46F, V46I, V46G, V46Y, V46L, V46A, V46P, V46W, V46M, V46S, V46T, V46N oder V46Q ausgewählte Mutation codiert.

9. Konstrukt gemäss jedem vorhergehenden Anspruch, worin das Konstrukt eine A4CT-DNA-Sequenz umfasst und ferner die Amyloid-Vorläuferprotein-Signalsequenz (APP-Reste 1 bis 17) unmittelbar stromaufwärts der A4CT-DNA-Sequenz zusammen mit Inserten von hydrophoben Resten zwischen codierenden Regionen für das Signalpeptid und A4CT codiert.

10. Konstrukt gemäss jedem vorhergehenden Anspruch, das einen Säugetier-Promotor umfasst, ausgewählt aus humanem APP (Amyloid-Vorläuferprotein); neurospezifischer Enolase der Ratte; humanem β-Actin; humanem PDGFβ (aus Blutplättchen gewonnener Wachstumsfaktor β); Maus-Thy 1; Prionprotein-Promotor der Maus; Prionprotein-Promotor des syrischen Hamsters; Ratten-Synapsin 1; humanem FMR1 (fragile X-mentale Retardierung 1); humanem Neurofilament low, middle; NEX-1; Maus-APLP2 (β-Amyloid-vorläuferartiges Protein 2); Ratten-α-Tubulin; Maus-Transferin; Maus-HMGCR (3-Hydroxy-3-methylglutaryl-Coenzym A-Reduktase) und basischem Maus-Myelinprotein.

11. Konstrukt gemäss Anspruch 1, das die Sequenz SPA4CT I45F (SEQ ID NO: 2) umfasst.

12. Konstrukt gemäss Anspruch 11, worin die DNA-Sequenz funktionsfähig mit Maus-Prion-Proteinpromotor verknüpft ist.

13. Konstrukt gemäss Anspruch 1, das die Sequenz APP695 I45F (SEQ ID NO: 4) umfasst.

14. Konstrukt gemäss Anspruch 13, worin die DNA-Sequenz funktionsfähig an syrischer Hamster-Prionprotein-Promotor geknüpft ist.

15. Nicht-humanes transgenes Säugetier, dessen Zellen ein Konstrukt gemäss jedem vorhergehenden Anspruch enthalten.

16. Transgenes Säugetier gemäss Anspruch 15, das ein Nagetier ist.

17. Transgenes Säugetier gemäss Anspruch 16, das eine Maus ist.

18. Säugetier-Wirtszelle, die das Konstrukt gemäss einem der Ansprüche 1 bis 14 exprimiert.

19. Vektor, der das Konstrukt gemäss einem der Ansprüche 1 bis 14 enthält.

20. Verfahren zur Durchmusterung auf Arzneistoffe, die die Ablagerung von βA4 hemmen, durch Verabreichung eines Testarzneistoffs an das transgene Säugetier von Anspruch 15, 16 oder 17 oder ein Zellkulturmedium, das die Säugetier-Wirtszelle von Anspruch 18 enthält, und Beobachten von Veränderungen in der APP-Expression und -Reifung, Histopathologie und/oder Verhaltensänderungen.

## Revendications

1. Construction comprenant une séquence d'ADN codant soit la protéine précurseur d'amyloïde humaine (APP) soit A4CT, dans laquelle la séquence d'ADN code la mutation 145F (numérotation par rapport à A4CT).

2. Construction selon la revendication 1, dans laquelle la séquence d'ADN est liée de manière opératoire à une séquence promotrice.

3. Construction selon la revendication 1, dans laquelle la construction code en outre pour une insertion d'une séquence de 1 à 10 résidus d'acide aminé dans le domaine transmembranaire.

4. Construction selon la revendication 3, dans laquelle l'insertion est située entre T48 et L49.

5. Construction selon la revendication 3 ou 4, dans laquelle les résidus pour une insertion sont choisis parmi F, I, G, Y, L, A, P, W, M, S, T, N et Q.

6. Construction selon l'une quelconque des revendications 3 à 5, dans laquelle l'insertion est longue de 2 à 6 résidus.

7. Construction selon la revendication 6, dans laquelle l'insertion est LV.

8. Construction selon l'une quelconque des revendications précédentes, dans laquelle la construction code en plus pour une mutation choisie parmi : V46F, V46I, V46G, V46Y, V46L, V46A, V46P, V46W, V46M, V46S, V46T, V46N, ou V46Q.

9. Construction selon l'une quelconque des revendications précédentes, dans laquelle la construction comprend une séquence d'ADN A4CT et code en outre pour la séquence signal de la protéine de précurseur d'amyloïde (résidus APP 1 à 17) immédiatement en amont de la séquence d'ADN A4CT conjointement avec les inserts de résidu hydrophobe entre le peptide signal et les régions codantes de A4CT.

10. Construction selon l'une quelconque des revendications précédentes, comprenant un promoteur de mammifère choisi parmi l'APP humaine (protéine précurseur d'amyloïde) ; l'énolase spécifique de neurone de rat ; l'actine β humaine ; le PDGFβ humain (facteur de croissance β dérivé des plaquettes) ; le Thy 1 murin ; le promoteur de protéine prion murine ; le promoteur de protéine prion de hamster syrien ; la synapsine 1 de rat ; la FMR1 humaine (retard mental 1 associé à l'X fragile) ; le neurofilament faible, moyen, humain ; NEX-1 ; la APLP2 murine (protéine de type précurseur d'amyloïde β 2) ; l'alpha tubuline de rat ; la transferrine murine ; la HMGCR murine (3-hydroxy-3-méthylglutaryl coenzyme A réductase) et la protéine basique de myéline murine.

11. Construction selon la revendication 1, comprenant la séquence SPA4CT I45F (SEQ ID n°2).

12. Construction selon la revendication 11, dans laquelle la séquence d'ADN est liée de manière opératoire au promoteur de protéine prion murin.

13. Construction selon la revendication 1, comprenant la séquence APP695 I45F (SEQ ID n°4)

14. Construction selon la revendication 13, dans laquelle la séquence d'ADN est liée de manière opératoire au promoteur de protéine prion de hamster.

15. Mammifère transgénique non humain, dont les cellules contiennent une construction selon l'une quelconque des revendications précédentes.

16. Mammifère transgénique selon la revendication 15, qui est un rongeur.

17. Mammifère transgénique selon la revendication 16, qui est une souris.

18. Cellule hôte mammifère exprimant la construction selon l'une quelconque des revendications 1 à 14.

19. Vecteur contenant la construction selon l'une quelconque des revendications 1 à 14.

20. Procédé de criblage de médicaments qui inhibent le dépôt de βA4, en administrant un médicament test au mammifère transgénique selon la revendication 15, 16 ou 17, ou un milieu de culture contenant la cellule hôte mammifère selon la revendication 18, et l'observation des changements dans l'expression et les modifications de APP, l'histopathologie et/ou les changements de comportement.
